(19) Europäisches Patentamt
European Patent Office
Office européen des brevets

(11) Numéro de publication: **0 089 860**
**A1**

(12) **DEMANDE DE BREVET EUROPEEN**

(21) Numéro de dépôt: **83400382.4**

(22) Date de dépôt: **25.02.83**

(51) Int. Cl.[3]: **C 07 D 453/02**
A 61 K 31/46

(30) Priorité: **05.03.82 FR 8203667**

(43) Date de publication de la demande:
**28.09.83 Bulletin 83/39**

(84) Etats contractants désignés:
**BE CH DE FR GB IT LI LU NL SE**

(71) Demandeur: **PHARMUKA LABORATOIRES**
**35, Quai du Moulin de Cage**
**F-92231 Gennevilliers(FR)**

(72) Inventeur: **Renault, Christian Louis Albert**
**10 Allée Réaumur**
**F-95150 Taverny(FR)**

(72) Inventeur: **Le Fur, Gérard Roger**
**35, rue du Progrès**
**F-92350 Plessis Robinson(FR)**

(74) Mandataire: **Leboulenger, Jean et al,**
**P C U K PRODUITS CHIMIQUES UGINE KUHLMANN**
**Service Propriéte Industrielle Tour Manhattan**
**F-92087 Paris La Défense 2 Cedex 21(FR)**

(54) **Isomère lévogyre de la méquitazine, son procédé de préparation et médicaments le contenant.**

(57) Composé, particulièrement utile comme médicament antihistaminique, de formula:

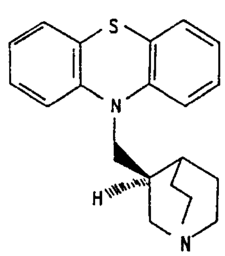

(1a)

dans laquelle la configuration absolue de l'atome de carbone en position 3 du cycle aza-1 bicyclo(2,2,2)octane est sinister (S).

EP 0 089 860 A1

Croydon Printing Company Ltd.

Isomère lévogyre de la Méquitazine, son procédé de préparation et médicaments le contenant

La présente invention a pour objet l'isomère lévogyre correspondant à l'[(aza-1 bicyclo(2,2,2)octyl-3)méthyl]-10 10H-phénothiazine racémique ou Méquitazine (dénomination commune internationale), son procédé de préparation, et son utilisation comme médicament, en particulier comme médicament antihistaminique.

La Méquitazine, qui répond à la formule :

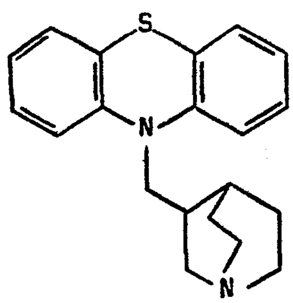

(I)

est un antihistaminique particulièrement utile pour le traitement des allergies. La Méquitazine a été décrite en particulier dans le brevet français n° 2 034 605.

La formule (I) comportant un atome de carbone asymétrique (carbone en position 3 du cycle aza-1 bicyclo(2,2,2)octane), la Méquitazine est dédoublable en deux inverses optiques ou énantiomères.

Le composé selon la présente invention, qui est l'énantiomère lévogyre, répond à la formule :

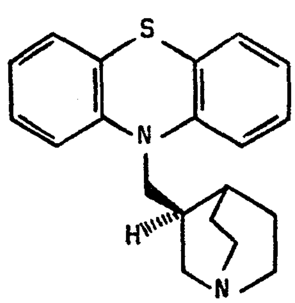

(Ia)

dans laquelle la configuration absolue de l'atome de carbone en position 3 du cycle aza-1 bicyclo(2,2,2)octane est sinister (S), et porte donc la

dénomination [(aza-1 bicyclo(2,2,2)octyl-3 (S)) méthyl]-10 10H-phénothiazine.

Le composé ($I_a$) peut être préparé par condensation du chlorométhyl-3 (S) [aza-1 bicyclo(2,2,2) octane] sur la phénothiazine, au sein d'un solvant inerte (par exemple un hydrocarbure aromatique comme le toluène ou le xylène, ou un solvant polaire tel que l'hexaméthylphosphorotriamide, ou encore un mélange de ces composés), en présence d'un agent de condensation alcalin, selon un procédé analogue à celui décrit dans le brevet français n° 2 034 605 pour la préparation de la Méquitazine à partir du chlorométhyl-3 [aza-1 bicyclo(2,2,2) octane] racémique et de la phénothiazine.

Le chlorométhyl-3 (S) [aza-1 bicyclo(2,2,2) octane] peut être obtenu par action du chlorure de thionyle sur l'[aza-1 bicyclo(2,2,2) octane-3 (S)] méthanol, selon un procédé identique à celui décrit par C.A. GROB et E. RENK, Helv. Chim. Acta, 37, (1954), 1689-1698 pour la préparation du chlorométhyl-3 [aza-1 bicyclo(2,2,2) octane] racémique à partir de l'[aza-1 bicyclo(2,2,2) octane-3] méthanol racémique.

L' [aza-1 bicyclo(2,2,2) octane-3 (S)] méthanol peut être obtenu par dédoublement de l'[aza-1 bicyclo (2,2,2) octane-3] méthanol racémique. Un tel dédoublement peut être réalisé par exemple en faisant réagir l'[aza-1 bicyclo(2,2,2)octane-3] méthanol racémique avec un acide optiquement actif ou un dérivé d'acide optiquement actif de façon à former un mélange de sels ou d'esters diastéréoisomères, puis en séparant lesdits diastéréoisomères à partir de leur mélange par des procédés classiques (par exemple par recristallisation) et en régénérant à partir de chaque diastéréoisomère la base ou l'alcool de départ sous forme optiquement pure, c'est-à-dire sous forme d'un énantiomère pur. Comme acides et dérivés d'acide optiquement actifs utilisables on peut citer ceux décrits dans S.H. WILEN, Tables of Resolving Agents and Optical Resolutions, University of Notre-Dame press, Notre-Dame, Indiana (1972).

Le composé ($I_a$) obtenu à l'état brut par le procédé décrit précédemment peut être purifié par des méthodes classiques, physiques (cristallisation, chromatographie) ou chimiques (formation de sel et régénération de la base par traitement du sel en milieu alcalin).

Le composé ($I_a$) sous forme de base libre peut être transformé en sel d'addition avec un acide minéral ou organique par action d'un tel acide au sein d'un solvant approprié.

Le composé ($I_a$) présente une activité antihistaminique supérieure à celle de la Méquitazine et une activité cholinolytique nettement inférieure à celle de la Méquitazine. Il en résulte que, à dose antihistaminique équiactive, le composé ($I_a$) produira moins d'effets cholinolytiques, donc moins d'effets secondaires (mydriase, sécheresse de la bouche, constipation) que la Méquitazine, ce qui est un avantage certain. Un tel résultat était parfaitement inattendu.

L'exemple suivant illustre l'invention sans la limiter.

EXEMPLE : Préparation de l'[(aza-1 bicyclo(2,2,2) octyl-3 (S)) méthyl]-10 10H-phénothiazine

1°) Préparation de l'[aza-1 bicyclo(2,2,2) octane-3 (S)] méthanol.

A 26,2 g d'[aza-1 bicyclo(2,2,2) octane-3] méthanol racémique dans 100 ml d'un mélange éthanol-$H_2O$ 80/20(parties en volume) on ajoute 27,9 g d'acide tartrique naturel (L). On porte à l'ébullition pous dissoudre les réactifs, puis laisse refroidir. Les cristaux obtenus sont séparés par filtration, lavés avec deux fois 20 ml d'un mélange éthanol-$H_2O$ 80/20, puis avec 50 ml d'oxyde diéthylique. On obtient ainsi 24 g de produit que l'on recristallise deux fois dans un mélange éthanol-$H_2O$ 80/20. Les 8,6 g de produit ainsi obtenus sont repris dans 400 ml de chloroforme et 15 ml d'une solution concentrée d'ammoniaque. La phase organique est séchée sur du sulfate de magnésium et le chloroforme est éliminé par évaporation sous pression réduite. Le résidu est repris dans 200 ml d'oxyde diéthylique, la solution est filtrée et évaporée à sec sous pression réduite. On obtient ainsi 3,35 g d'[aza-1 bicyclo(2,2,2) octane-3 (S)] méthanol, qui fond à 56-58°C et dont le pouvoir rotatoire spécifique (mesuré sur une solution à 3 % du produit dans une solution aqueuse N d'acide méthanesulfonique) est

$$\alpha_D^{24} = -65,8°$$

La pureté optique du produit obtenu, déterminée par la méthode de J.A. DALE et Coll. J. Org. Chem. 34, (1969), 2543, est 99 %.

2°) Préparation du chlorométhyl-3 (S) [aza-1 bicyclo(2,2,2) octane].

Par action du chlorure de thionyle sur l'[aza-1 bicyclo(2,2,2) octane-3 (S)] méthanol au sein du chloroforme on prépare le chlorhydrate de chlorométhyl-3 (S) [aza-1 bicyclo(2,2,2) octane] , qui fond à température supérieure à 260°C et dont le pouvoir rotatoire spécifique (mesuré sur une solution aqueuse à 1 % du produit) est

$$\alpha_D^{24} = - 58,7°$$

En traitant le chlorhydrate ci-dessus en milieu aqueux par une base minérale telle que l'hydroxyde de sodium, puis en extrayant le produit formé avec de l'oxyde diéthylique et en éliminant l'oxyde diéthylique par évaporation, on obtient le chorométhyl-3 (S) [aza-1 bicyclo(2,2,2) octane].

3°) Préparation de l'[(aza-1 bicyclo(2,2,2) octyl-3 (S)) méthyl]-10 10H-phénothiazine.

On place sous atmosphère d'azote 6 g de phénothiazine, 2,9 g de tertiobutylate de potassium, 3 ml d'hexaméthylphosphorotriamide et 30 ml de xylène anhydre. On porte le mélange à l'ébullition, distille 10 ml de solvant et ajoute, en l'espace d'une heure, une solution de 2,4 g de chlorométhyl-3 (S) [aza-1 bicyclo(2,2,2) octane] dans 30 ml de xylène anhydre. On maintient l'ébullition pendant une heure, refroidit le mélange réactionnel, ajoute 20 ml d'eau, sépare la phase aqueuse par décantation et l'extrait avec trois fois 20 ml de chloroforme. La phase chloroformique est séchée sur du sulfate de magnésium, puis le chloroforme est éliminé par évaporation sous pression réduite. On obtient ainsi 10 g d'un produit brut qui est fixé sur une colonne de gel de silice. On élue avec un mélange de 95 parties en volume de toluène et 5 parties en volume de diéthylamine. Les fractions contenant le produit recherché (ces fractions sont repérées à l'aide d'un test par chromatographie en couche mince sur silice, la présence du produit recherché dans une fraction étant mise en évidence dans ce test par une tache

située au même endroit que celle donnée par le produit de référence Méquitazine) sont évaporées. On récupère ainsi 5 g de produit que l'on fixe à nouveau sur une colonne de gel de silice. On élue avec un mélange de 90 parties en volume de chloroforme, 5 parties en volume de méthanol et 5 parties en volume d'acide acétique. On évapore les fractions contenant le produit recherché (fractions repérées comme indiqué précédemment). On obtient ainsi 2,4 g de produit que l'on dissout dans l'eau. La solution aqueuse est alcalinisée par addition d'une solution concentrée d'ammoniaque et l'insoluble est extrait au chloroforme. La phase chloroformique est séchée sur du sulfate de magnésium et évaporée sous pression réduite. On obtient ainsi 1,15 g de produit que l'on recristallise dans l'acétone, ce qui fournit 0,7 g d'[(aza-1 bicyclo (2,2,2) octyl-3 (S))méthyl]-10 10H-phénothiazine, qui fond à 139-140°C et dont le pouvoir rotatoire (mesuré sur une solution à 1 % du produit dans l'éthanol) est

$$\alpha_D^{23} = -39,8°$$

La pureté optique du produit obtenu, déterminée par la méthode calorimétrique décrite par C. FOUQUEY et J. JACQUES, Tetrahedron, <u>23</u>, (1967), 4009, est 97,5 %.

## PROPRIETES PHARMACOLOGIQUES

L'activité antihistaminique des composés [composé ($I_a$) et Méquitazine] a été évaluée en mesurant leur affinité pour les récepteurs $H_1$ de l'histamine. Cette affinité a été mesurée par l'aptitude des produits à déplacer la pyrilamine tritiée ($^3H$ pyrilamine) de son site de liaison et est exprimée par une valeur $IC_{50}$ qui est la concentration de produit (en nanomoles par litre) nécessaire pour obtenir une inhibition de 50 % de la liaison de la $^3H$ pyrilamine. Les produits ont été testés selon le protocole de V.T. TRAN et Coll., Proc. Nat. Acad. Sci., (USA), 75, 6290 (1978), en utilisant des membranes de cerveau de cobaye.

L'activité cholinolytique des composés a été évaluée en mesurant leur affinité pour les récepteurs muscariniques de l'acétylcholine. Cette affinité a été mesurée par l'aptitude des produits à déplacer

le benzilate de quinuclidinol-3 tritié ($^{3}H$ QNB) de son site de liaison et est exprimée par une valeur $IC_{50}$ qui est la concentration de produit (en nanomoles par litre) nécessaire pour obtenir une inhibition de 50 % de la liaison du $^{3}H$ QNB. Les produits ont été testés selon le protocole de H.I. YAMAMURA et Coll., Proc. Nat. Acad. Sci., (USA) 71, 1725, (1974), en utilisant des membranes de striatum de cerveau de rat.

Les résultats obtenus sont rassemblés dans le tableau suivant :

| Composés | $IC_{50}$ (nM/l) | |
|---|---|---|
| | $^{3}H$ - QNB | $^{3}H$-pyrilamine |
| Méquitazine racémique | 30 | 37 |
| Composé ($I_a$) | 120 | 27 |

Le composé de formule ($I_a$) est donc 1,4 fois plus antihistaminique et 4 fois moins cholinolytique que la Méquitazine racémique, c'est-à-dire que, à doses équiactives, le composé ($I_a$) est environ 5 fois moins cholinolytique que le racémique.

Les toxicité aiguës du composé ($I_a$) et de la Méquitazine déterminées chez la souris sont sensiblement identiques.

## UTILISATION THERAPEUTIQUE

Le composé de formule ($I_a$) et ses sels avec un acide pharmaceutiquement acceptable peuvent être utilisés en thérapeutique humaine comme matière active de médicament, en particulier de médicament antihistaminique.

Un tel médicament contient, outre la matière active, un véhicule pharmaceutiquement acceptable tel que ceux communément utilisés

dans le domaine pharmaceutique et peut se présenter sous forme de comprimés, capsules, gélules, suppositoires, solution ingérable ou injectable, etc...

La posologie dépend des effets recherchés et de la voie d'administration utilisée. Par exemple, par voie orale, elle se situe en général pour un adulte entre 10 et 200 mg par jour du substance active.

Revendications de brevet

1. Composé de formule :

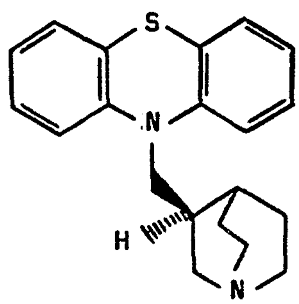

(Ia)

dans laquelle la configuration absolue de l'atome de carbone en position 3 du cycle aza-1 bicyclo(2,2,2)octane est sinister (S).

2. Procédé de préparation du composé selon la revendication 1, caractérisé en ce que l'on condense, au sein d'un solvant inerte, en présence d'un agent de condensation alcalin, le chlorométhyl-3 (S) [aza-1 bicyclo(2,2,2)octane] avec la phénothiazine.

3. Médicament, particulièrement utile comme médicament antihistaminique, contenant une substance active et un véhicule pharmaceutiquement acceptable, caractérisé en ce que la substance active est le composé selon la revendication 1 ou un sel de ce composé avec un acide pharmaceutiquement acceptable.

Office européen des brevets

# RAPPORT DE RECHERCHE EUROPEENNE

0089860
Numéro de la demande

EP 83 40 0382

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| X | ANNALES PHARMACEUTIQUES FRANCAISES, vol. 33, no. 6-7, 1975, pages 345-354, Paris, FR. A. UZAN et al.: "Effets de différents hétérocycles sur l'incorporation de 5-hydroxytryptamine et de noradrénaline par une préparation de cerveau de Rat" * Article en entier; spécialement composé LM 209 * --- | 1-3 | C 07 D 453/02<br>A 61 K 31/46 |
| X | "Methoden der organischen Chemie", Houben-Weyl, vol. IV, partie 2, 1955, "Allgemeine chemische Methoden", Georg Thieme Verlag, Stuttgart, DE. * Pages 526-528 * --- | 1,2 | |
| X,D | FR-A-2 034 605 (SOGERAS) * Exemple II; revendications * ----- | 1-3 | DOMAINES TECHNIQUES RECHERCHES (Int. Cl. 3)<br>C 07 D 453/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 13-06-1983 | NUYTS A.M.K.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant